# EUROPEAN PATENT SPECIFICATION

(11) **EP 3 870 037 B1**
(45) Date of publication and mention of the grant of the patent: **23.11.2022**
(21) Application number: 19797769.7
(22) Date of filing: 22.10.2019
(51) Int. Cl.: A61B 5/246

(54) **HEAD-MOUNTABLE APPARATUS**
KOPFMONTIERBARE VORRICHTUNG
APPAREIL POUVANT ÊTRE MONTÉ SUR LA TÊTE

(30) Priority: 23.10.2018 FI 20185893
(43) Date of publication of application: 01.09.2021
(73) Proprietor: Megin Oy, 00531 Helsinki (FI)
(72) Inventor: LAINE, Pasi, 00670 Helsinki (FI); HELLE, Liisa, 00150 Helsinki (FI); NENONEN, Jukka, 02400 Kirkkonummi (FI)
(74) Representative: Papula Oy
(86) International application number: PCT/FI2019/050748
(87) International publication number: WO 2020/084194

(56) References cited:
- US-A1- 2010 219 820
- US-A1- 2014 121 491
- US-A1- 2016 174 862
- US-A1- 2017 165 485
- US-A1- 2017 296 295
- US-A1- 2018 169 428
- US-B1- 9 791 536

## Description

### FIELD

The present disclosure relates to measurement of magnetic brain response. In particular, the present disclosure relates to magnetoencephalography measurement devices for measuring magnetic brain response to auditory stimuli.

### BACKGROUND

Magnetoencephalography (MEG) is a functional neuroimaging technique for mapping brain activity by recording magnetic fields produced by electrical currents which occur naturally in the brain. For this purpose, very sensitive magnetometers are required to distinguish useful signals from noise arising from sources external to the brain. Currently, the most common magnetometer used is SQUID (superconducting quantum interference device), which typically requires cryogenic cooling down to very low operating temperatures. Consequently, such MEG devices for measurement of magnetic brain response have bulky construction and typically involve a rigid helmet-like construction integrated in the measurement system for positioning of the head, and consequently the brain, of the test subject. The system may, for example, be comprise a gantry or a chair, where the helmet-like construction is integrated.

While the aforementioned configuration allows precise enough measurement to record the magnetic fields of the brain regardless of the surrounding noise, it has various drawbacks. For example, the helmet-like configuration described above may not necessarily be used for two different test subjects, if the sizes of their heads are different. In particular, this may prevent the same device from being used for both adults and children. In addition, the helmet-like configuration may restrict the use of the device in case the test subject experiences fear or anxiety from their head being engulfed in the measurement device.

Systems for measuring magnetic brain responses to auditory stimuli are known from US-2014/121491-A1, US-2010/219820-A1, US-9791536-B1 and US-2016/174862-A1. Earphone-shaped measurement/stimulation systems are known from US-2017/165485-A1, US-2018/169428-A1 and US-2017/296295-A1.

### OBJECTIVE

An objective is to eliminate or alleviate at least some of the disadvantages mentioned above.

In particular, it is an objective to provide an apparatus that provides a precise enough signal to measure magnetic brain response of a test subject, while not requiring the head of the test subject to be enclosed in a measurement helmet. The signal should not only be precise enough to overcome the external noise emerging from sources positioned outside the head of the test subject but also such that brain response specifically related to one or more auditory stimuli can be determined. Additionally, it is an objective to provide an apparatus which can be used to measure magnetic brain response of two test subjects, such as an adult and a child, having different head sizes. Furthermore, it is an objective to provide an apparatus which can follow the movement of the head, simplifying calibration of the apparatus and improving accuracy, in particular with test subjects having difficulty in remaining stationary during measurement.

### SUMMARY

By test subject, it is intended here a human being whose brain response is to be measured. The brain response is measured from the head of the test subject. By brain response to one or more auditory stimuli it is intended here the electric current and/or magnetic field originating in the brain of a test subject. Consequently, magnetic brain response corresponds to the magnetic field generated by the brain in response to one or more auditory stimuli. The magnetic brain response to auditory stimuli is below referred to also as the target signal.

Below, it is repeatedly referred to positioning or mounting parts of a measurement apparatus on an auditory cortex. This corresponds to positioning or mounting the corresponding part of the measurement apparatus on the area on the surface of the head of the test subject, where the measurement of magnetic response originating in the auditory cortex can be performed. For the right auditory cortex, this may be considered to correspond to the planar projection of the auditory cortex on the surface of the right side of the head. For the left auditory cortex, this may be considered to correspond to the planar projection of the auditory cortex on the surface of the left side of the head.

According to a first aspect, a head-mountable apparatus (below also "apparatus") for measuring magnetic brain response to auditory stimuli is disclosed. The apparatus may be formed as a head set and it is adapted to be worn on a head of a test subject. The apparatus comprises two separate cases, each of the cases adapted to be mounted on one side of the head to allow measurement of brain response to auditory stimuli on that side of the head. Specifically, the cases are adapted to be mounted on the auditory cortices of the brain allowing, on one hand, close enough proximity to one or more signal sources in the brain generating response to auditory stimuli, and, on the other hand, far enough distance to other signal sources in the brain, i.e. those not associated with generating the target signal, so that any magnetic fields generated by these sources do not overwhelm the target signal. A first case is adapted to be mounted on the head on the right auditory cortex for measuring the brain response of the right auditory cortex and a second case is adapted to be mounted on the head on the left auditory cortex for measuring the brain response of the left auditory cortex. Consequently, both cases are adapted to be positioned a little above the ears of the test subject, for example resting on the ears. While not necessarily covering the ears, the cases may be formed as earphones positioned on the auditory cortices. By mounting the cases on the head and specifically on the auditory cortices, the measurement region of the apparatus can be focused on the auditory cortices so that the target signal can be extracted. The measurement region of the apparatus can thereby also be limited specifically to the auditory cortices so that the apparatus is specifically directed to measure the signals generated by the auditory cortices. This allows other signals than the target signal originating from the brain to be abated or be reduced below negligible level from the measurement. It is noted that when a case is positioned on an auditory cortex, it is meant here that the case is positioned on the outer surface of the head corresponding to the location to the auditory cortex so that the apparatus can be used external to the body, i.e. without penetrating the skin of the test subject. Alternatively, it may be said that the case is positioned over or above the auditory cortex, as seen from the direction substantially perpendicular to the surface of the head of the test subject that is covered by the case. This direction is generally substantially horizontal when the apparatus is in use.

The apparatus comprises two separate sets of optically pumped magnetometers (OPM) for measuring magnetic brain response to auditory stimuli, each set housed in one of the cases. While each set may comprise any number of OPMs, with a natural minimum of one OPM, increasing the number of OPMs may be used to improve accuracy and/or spatial resolution of the apparatus. OPM technology in itself is already known. Typically, it involves structures with chambers of gas or vapor, for example of alkali atoms. To make the chambers to function as magnetic field sensors methods such as spin resonance detection, e.g. electron-spin resonance detection can be used. The material in the chambers can be polarized by optical pumping, for example by a laser beam, which may have a certain polarization such as a circular polarization. A magnetic field, such as that produced by the target signal, changes the orientation of spins of the material in the chambers, which can, in turn, be probed to determine the magnetic field. This probing can also be done optically, for example by a second laser beam.

Incorporating OPMs in the present apparatus provides various effects. Firstly, it allows the apparatus to function as a measurement device in room temperature, which further allows the apparatus to function without cryogenic cooling apparatus. Among other benefits, this improves the mobility of the apparatus since it can now follow the movement of the head of the test subject, consequently also allowing free movement for the head. Additionally, the OPMs can be positioned tightly against the head of the test subject to minimize the source-to-sensor distance, e.g. the distance between an auditory cortex and the OPMs adapted to measure the signal from the auditory cortex. This can be exploited when the OPMs are positioned on the auditory cortices to maximize the amplitude of the signal from the auditory cortices that can be measured by the OPMs, allowing high enough precision so that the measurement of the target signal can actually be performed. Conversely, noise from sources external to the head and/or other sources in the brain can be decisively reduced. When the cases are positioned on the auditory cortices, the OPMs may be positioned in the cases so that the measurement area of the apparatus is limited to the auditory cortices, thereby focusing the measurement on the target signal and abating signals originating from other sources in the brain. Since all of the OPMs of the apparatus can be positioned on the auditory cortices, effect of internal noise from the brain can be minimized, the internal noise being magnetic signal originating in the brain other than the target signal. The position of the OPMs housed in a case may be fixed with respect to the case so that the position and orientation of the case directly corresponds to the position and orientation of the OPMs housed in the case.

In addition to the above, the apparatus comprises at least one output interface for transmitting measurement data from the apparatus. This may comprise a wireless and/or a wired interface. The output interface can also be adapted for transmitting data from one or more additional sensors such as reference sensors. Conversely, the apparatus may comprise at least one input interface for transmitting information, such as control instructions, to the apparatus.

The apparatus as described herein can be adapted for clinical use. In particular, the apparatus being able to measure the magnetic brain response specifically corresponding to auditory stimuli allows it to be used for various purposes such as potentially diagnosing and/or guiding therapy for autism. Since the apparatus can move with the head and since it can be made small and light-weighted, it can be made comfortable for the user to wear. This may be particularly relevant, when the test subject is a child or suffers from anxiety. It is noted that ease of use for the apparatus is particularly important since a single measuring session can take, for example, 15 minutes but it may take also a longer time. Consequently, the apparatus has been adapted for use for an extended period of time, corresponding to at least 15 minutes.

The same apparatus can be adapted to be used for measurement of magnetic brain response for heads of different size and/or shape, such as the head of a child and the head of an adult. For example, the apparatus may be adapted to be used for two heads having circumferences that differ from each other by at least 5 or 10 centimeters but the construction of the apparatus allows the apparatus to be even adapted to be used for two heads having circumferences that differ from each other by at least 20 centimeters.

In an embodiment, the first case covers the right auditory cortex and the second case covers the left auditory cortex. This allows the set of OPMs in each of the cases to be spread on the auditory cortex to improve accuracy and/or spatial resolution of the measurement. The set of OPMs in either or both of the cases can be spread on the whole auditory cortex.

In an embodiment, the apparatus comprises a connector mechanically coupling the first case to the second case. This allows the cases to be maintained together, for example for aligning the apparatus with respect to the head. The connector may be a band, for example an arched band. The width of the connector may be the same or smaller in comparison to the width cases, for example the smaller one in case there is any difference. This allows a light connection between the cases so that the head is not excessively covered. In a further embodiment, the connector is adapted to extend across the top of the head for supporting the weight of the first case and the second case when the head is upright. In addition to ease-of-use and aid in accurate positioning of the OPMs on the auditory cortices, this improves the correspondence in construction between the apparatus and a set of conventional audio headphones. This may have notable advantages for being able to appropriately use the apparatus with some test subjects, for example autistic ones, who otherwise may suffer from anxiety using more restrictive constructions. In another further embodiment, the connector is flexible so that it presses the first case and the second case against the head for measurement by OPMs. As it is important for performing the measurement that a tight contact between the measurement apparatus and the surface of the head is obtained, the connector may be adapted to be flexible enough so that the contact between the cases and the head for measurement is maintained by the connector. In another further embodiment, the length of the connector between the first case and the second case is adjustable. This allows the cases, and correspondingly the OPMs in each of the cases, to be accurately positioned for heads of different sizes.

In an embodiment, the apparatus comprises one or more pointers for aligning the apparatus with respect to one or more anatomical landmarks of the head. This allows improving accuracy in positioning the apparatus, in particularly the sets of OPM sensors. At least some of the pointers may be configured to indicate the position of either or both the cases, and correspondingly the position of the set of OPMs housed in the case, with respect to the head of the test subject. By position, it is meant here a single point, such as a center of mass, describing the overall position of the case. Such a pointer for indicating position may be fixed with respect to the case whose position it is configured to indicate. On the other hand, at least some of the pointers may be configured to calibrate the orientation, in particular the pitch, of either or both the cases with respect to the head of the test subject. Correspondingly, such a pointer for indicating orientation, in particular the pitch, may be of adjustable length. It is noted that, in the context of this disclosure, "pitch" can be defined as the degree of rotation about the axis intersecting the first case and the second case.

In a further embodiment, the one or more anatomical landmarks comprise the nasion and/or a pre-auricular point. These locations of the head have been found to provide quickly realizable calibration in practice, while at the same time providing robust calibration accuracy. In addition, the points are anatomically sufficiently well-defined for different shapes and sizes of head so that the calibration can be efficiently performed with a single apparatus. Nasion, in particular, can be used as an anatomical landmark for a pointer configured to calibrate the pitch of the apparatus and/or the cases. A pre-auricular point, in particular, can be used as an anatomical landmark for a pointer configured to calibrate the position of a case or a set of OPMs. In another further embodiment, the one or more pointers comprises a tip and/or a hole for locating the one or more landmarks. This allows speed and accuracy in placement of the pointer. In another further embodiment the first case and/or the second case forms at least one of the one or more pointers. This can be used, for example, when an anatomical landmark corresponding to this pointer is an auricular point or a pre-auricular point. With the structure of the case being rigid, this allows the case to be directly aligned with the head. In another further embodiment, the apparatus comprises a connector mechanically coupling the first case with the second case, wherein at least one of the one or more pointers (hereafter also "nasal pointer") is coupled to the connector and adapted to extend on the forehead of the head towards the nose of the head. This allows the pointer to support the apparatus on the head. Additionally, it can be used for speed and accuracy in placement of the pointer, in particular when this nasal pointer is configured to calibrate the orientation, such as the pitch, of either or both the cases with respect to the head of the test subject. It has been found that the nasion can be used as an anatomical landmark for this pointer to provide quickly realizable calibration in practice, while at the same time providing robust calibration accuracy. In a yet further embodiment, the length of extension from the connector of the at least one pointer coupled to the connector is adjustable. This allows the nasal pointer to be adjusted for the size of the head of the test subject. The apparatus may comprise a mechanical scale, for example in the nasal pointer, for determination of the position of the nasal pointer. Alternatively or in addition, the apparatus may be adapted for automated detection of the length of extension of the nasal pointer.

In an embodiment, the apparatus is adapted to use one or more speakers and/or one or more ducts for directing auditory stimuli into an ear of the head. This allows, for example, the auditory stimuli to be introduced in a fixed manner with respect to the apparatus. The one or more speakers and/or one or more ducts may be comprised in the apparatus or they may be externally provided.

In an embodiment, the apparatus comprises one or more accelerometers for measuring movement of the head. This allows further improvement in calibration of the measurement, consequently making it possible to improve the accuracy of the measurement. Using the one or more accelerometers, the apparatus may also be adapted to compensate for movement-related interference in the measurement, for example when the head of the test subject moves.

In an embodiment, each of the first case and the second case comprises at least one optically pumped magnetometer adapted to function as a gradiometer for measuring magnetic brain response to auditory stimuli. Using gradiometric measurement allows improved detection of brain response in auditory cortices. This may be done by using two or more OPMs, each individual OPM providing a magnetometer signal, i.e. a signal corresponding to magnetic field strength. The apparatus is then adapted to provide a gradiometer signal, by means known to a person skilled in the art, for example by a physical arrangement of a measurement circuit including the OPMs adapted to function as a gradiometer and/or signal processing of the measurement signal. As an alternative, if available, an OPM which by construction is adapted to function as a gradiometer, may be used.

It is to be understood that embodiments described above may be used in any combination with each other and the aspect described above. Several of the embodiments may be combined together to form a further embodiment.

### BRIEF DESCRIPTION OF THE DRAWINGS

The accompanying drawings, which are included to provide a further understanding and constitute a part of this specification, illustrate embodiments and together with the description help to explain the principles of the invention. In the drawings:
**Fig. 1** illustrates a head-mountable apparatus according to an embodiment in a perspective view,
**Fig. 2** illustrates a head-mountable apparatus according to an embodiment on the head of a test subject in a perspective view,
**Fig. 3** illustrates a head-mountable apparatus according to another embodiment on the head of a test subject in a perspective view, and
**Fig. 4** illustrates anatomical landmarks of a human head in a side view.

Like references are used to designate equivalent or at least functionally equivalent parts in the accompanying drawings.

### DETAILED DESCRIPTION

The detailed description provided below in connection with the appended drawings is intended as a description of the embodiments and is not intended to represent the only forms in which the embodiment may be constructed or utilized. However, the same or equivalent functions and structures may be accomplished by different embodiments.

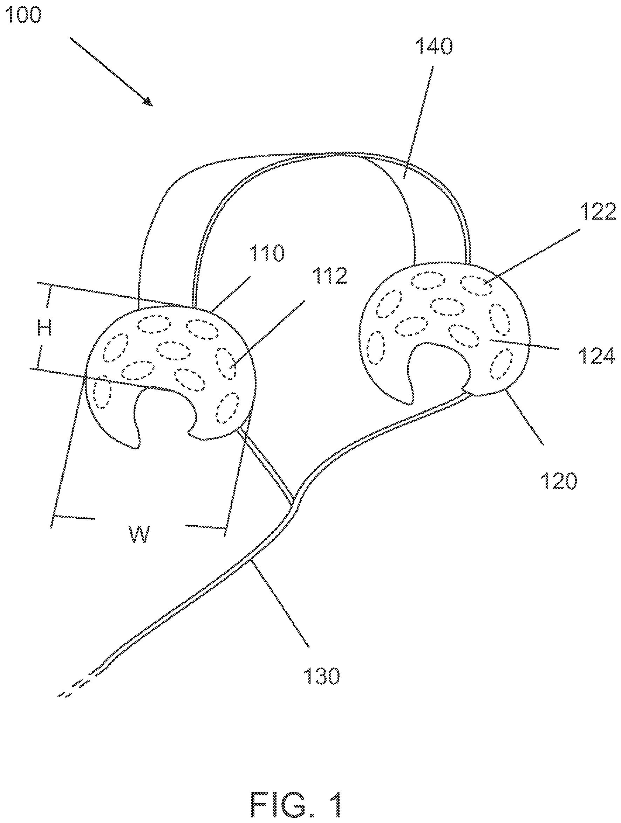

Figure 1 shows an example of a head-mountable apparatus (below also "apparatus") 100 for magnetoencephalography. The apparatus 100 can be configured to measure magnetic brain response to auditory stimuli, for example for diagnosing or guiding therapy for autism. The apparatus 100 comprises a first case 110 and a second case 120, each adapted to house one or more OPMs 112, 122. Each of the cases 110, 120 is adapted to be supported on the head of a test subject. One of the cases 110 is adapted to be supported on the right auditory cortex of the brain of the test subject and one of the cases 120 is adapted to be supported on the left auditory cortex of the brain of the test subject. One case 110, 120 may be positioned on one auditory cortex so it is enough for the apparatus 100 to comprise exactly two such cases 110, 120 for the measurement of target signal. The cases 110, 120 can be symmetrical with respect to each other, in particular left-right symmetrical.

Either or both of the cases 110, 120 may be adapted to be supported on an ear of the test subject. For this purpose, a case 110, 120 may comprise a cavity, for example a half-open cavity such as an essentially U-shaped cavity. Either or both of the cases 110, 120 may be adapted to expose the helix of the corresponding ear. Either or both of the cases 110, 120 may comprise or be made of plastic.

The apparatus 100 comprises two sets of OPMs 112, 122, each housed in one case 110, 120. The OPMs 112, 122 may be fixed within the cases 110, 120, in contrast to equipment where sensors are adapted to be moved during measurement. Also, the position and orientation of the OPMs 112, 122 can be fixed within each case 110, 120. The OPMs 112, 122 in both sets can be configured to measure magnetic brain response to auditory stimuli, for example for diagnosing or guiding therapy for autism. The OPMs 112, 122 may be enclosed in the cases 110, 122 or they may, at least partially, extend from the cases 110, 122. The number of OPMs may be one, two or larger and it can be increased to increase accuracy and/or spatial resolution of the measurement of the target signal. Consequently, each set may easily comprise ten or more OPMs 112, 122. The number of OPMs 112, 122 in the two sets may be the same or it may be different. The OPMs 112 in one case 110 may be symmetrically positioned with respect to the OPMs 122 in the other case 120, in particular with left-right symmetry. Some or all of the OPMs 112, 122 may be adapted to function as gradiometers, for example by being positioned in a pair-wise arrangement in gradiometer configuration. Due to the OPM sensors, the apparatus 100 is adapted for measurement at room temperature, or in any reasonable temperature for clinical operation, e.g. an ambient temperature of 0-50 degrees of Celsius, without cooling equipment such as a cryocooler. The OPM sensors 112, 122 themselves can be adapted for measurement at any of these ambient temperatures, for example by using their own micro coolers, if necessary.

Depending on the specific type of an OPM sensor, the temperature of the OPM 112, 122 may be over 100 degrees Celsius, for example 150 degrees Celsius. Therefore, the apparatus 100 may comprise thermal insulation for allowing the apparatus to be used with test subjects regardless of the heat generated in the OPMs, e.g. by reducing the temperature of the apparatus 100 below 40 or 50 degrees of Celsius on any surface adapted for contact with the head. This thermal insulation may be arranged in either or both of the cases 110, 120, for example on the surface of the case 110, 120 adapted to contact the side of the head.

In addition to sensors used directly for measurement, the apparatus 100 may comprise additional sensors such as reference sensors, for example for error correction and/or noise removal. These additional sensors may comprise OPM sensors. The additional sensors may be located within a case 110, 120 or external to it. Even sensors separate to the apparatus 100 may be used for any of the aforementioned purposes.

For contact with the side of the head, either or both of the cases 110, 120 may comprise or be coupled with a contact surface 124. The contact surface 124 can be adapted to contact the side of the head on an auditory cortex. It may comprise a layer of thermal insulator for allowing the apparatus to contact the head regardless of heat generated in the OPMs 112, 122. The contact surface may comprise, for example, a layer of plastic or fabric. The contact surface may be releasable to allow to removal and replacement by another contact surface. This allows the contact surface to be replaced for hygiene. Regardless of whether the contact surface 124 is present or not, the OPMs 112 in either or both cases 110, 120 may be adapted to be located within one centimeter from the surface of the head of the test subject when the apparatus 100 is in use.

Either or both of the cases 110, 120 may cover the whole auditory cortex. This allows also the OPMs 112, 122 in the corresponding case 110, 120 to be spread on the whole auditory cortex. For this purpose, the width W of a case 110, 120 can be at least 8 centimeters. To allow freedom in positioning the OPMs 112, 122, the width W can be 10 centimeters or more. Similarly, the height H of a case 110, 120, measured from a part of the case 110, 120 adapted to rest on the ear of a test subject, can be at least 4 or 6 centimeters to allow measurement across the whole auditory cortex. It is noted that since an auditory cortex has an elongated shape, it is enough for the maximum cross section of a case 110, 120 to have length of at least 8-10 centimeters. Correspondingly, to cover the auditory cortex, the maximum distance between two OPMs within a case 110, 120 should be 8-10 centimeters or more.

The apparatus 100 comprises an output interface 130 for transmitting measurement data from the apparatus 100. The output interface 130 may be wired interface, as pictured, or a wireless interface. The output interface 130 may comprise two separate interfaces for the two sets of OPMs 112, 122 or the apparatus 100 can be adapted so that the measurement data from both sets of OPMs 112, 122 can be transmitted from the apparatus 100 through one output interface 130.

The apparatus 100 may comprise a processor or a controller configured to process the measurement signal obtained from the OPMs 112, 122 before it is transmitted from the apparatus 100 or the apparatus 100 may be adapted for the measurement data to be transmitted directly from the OPMs 112, 122 without pre-processing. In any case, the apparatus 100 may comprise a converter for converting the measurement signal into a digital signal before transmitting it from the apparatus 100. For example, either or both of the cases 110, 120 may house such a converter.

The apparatus 100 may comprise a connector 140 adapted to mechanically couple the two cases 110, 120. The connector may be flexible or semi-flexible and it may be adapted to press the cases 110, 120 against the head of the test subject so that the measurement can be performed. This is particularly important for a measurement using OPMs 112, 122, where weak measurement accuracy may be significantly improved by reducing the distance between the OPMs 112, 122 and the target of measurement, in this case the head of the test subject. The apparatus 100 may be adapted for the OPMs, or their measuring surface in particular, to be positioned within 2-20 millimeters or even within 1-10 millimeters from the surface of the head of the test subject. For example, the connector 140 may be adapted to exert a force on the cases 112, 122 exceeding a threshold force for holding the cases 110, 120 stationary on the head of the test subject irrespective of gravity. Alternatively or in addition, the connector 140 may be adapted to be supported on the head of the test subject, e.g. on the scalp, to hold the cases 110, 120 stationary on the head of the test subject irrespective of gravity. For this purpose, the connector 140 may extend across the head, for example across the top of the head. The connector 140 may be arched, for example corresponding to a substantially U-shaped arc in shape. The connector 140 may be a band. It may be limited in size so as to cover only part of the surface of the head above the cases 110, 120, for example less than 50 percent or even less than 10 or 20 percent of the surface of the head above the cases 110, 120. Conversely, regardless of whether the connector 140 is included or not, the apparatus 100 may be adapted to expose more than 50 percent or even more than 80 or 90 percent of the surface of the head above the ears of the test subject. This allows ease of use and may allow the use of the apparatus for measurement with various groups of test subjects that cannot use a more confining apparatus. The connector 140 may be adapted for remaining stationary on the head during measurement. The apparatus 100 can also be adapted to expose the helix of one or both of the ears of the test subject.

For the purposes described above, the connector 140 may be adapted to flex in lateral dimension, i.e. the dimension parallel to an axis intersecting the cases 110, 120, to press the cases 110, 120 against the ears. However, the connector 140 and/or the apparatus 100 may also be adapted to be substantially rigid in either or both of the perpendicular dimensions, i.e. in the vertical dimension and/or in the back-forward dimension. In this manner, the connector 140 may be adapted for rigid fixing with respect to the first case 110 and the second case 120 so that it remains stationary with respect to the cases 110, 120 upon rotation of the apparatus 100, in any or all directions, particularly with respect to the axis intersecting the cases 110, 120, i.e. the axis corresponding to the pitch of the apparatus 100. For the above purpose, the connector 140 may be rigidly fixed with respect the cases 110, 120 or the apparatus 100 may comprise one or more tighteners for rigidly fixing the connector 140 with respect to the cases 110, 120. In any case, the connector 140 may be sufficiently rigid to maintain its shape in rest regardless of its orientation. As an example, the connector 140 may comprise or be made of plastic.

Since the two sets of OPMs 112, 122 in the two separate cases 110, 120 are adapted to measure the target signal from separate auditory cortices, it is not necessary to know or maintain their position with respect to each other. Correspondingly, the connector 140 may be adapted so that the length of the connector 140 between the two cases 110, 120 is adjustable. For this purpose, the connector 140 may be adapted for its total length to be changed and/or the connector 140 may be adapted for its fixing point with either or both of the cases 110, 120 to be adjustable along the length of the conductor 140. By making the length of the connector 140 between the two cases 110, 120 to be adjustable allows the apparatus 100 be adapted for differently sized heads.

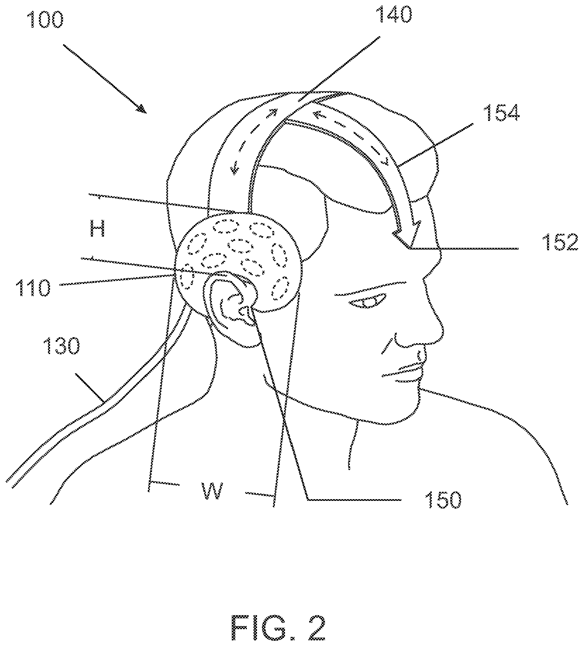

Figure 2 shows another example of the apparatus 100, where the apparatus 100 is mounted on the head of a test subject 200. In this example, the apparatus 100 comprises a set of pointers 150, 152 for aligning the apparatus with respect to the head. A pointer 150, 152 is adapted to indicate the alignment of the apparatus 100 with respect to the head so that the apparatus 100 can be calibrated for measurement. In particular, the pointer 150, 152 can be adapted to be aligned with an anatomical landmark of the head. Since the apparatus 100 can follow the movements of the head, the position and orientation of the apparatus 100 can remain constant with respect to the position and orientation of the head and, correspondingly, the source of the target signal, i.e. the auditory cortices. The construction of the apparatus 100 allows this calibration to be performed in single one-off calibration. Moreover, the construction together with the use of pointers 150, 152 adapted to align the apparatus 100 with respect to one or more anatomical landmarks allows the calibration to be performed quickly and accurately. The pointers 150, 152 may be adapted for accuracy of one centimeter or better on the surface of the head but also for much better accuracy, for example, for an accuracy two or five millimeters or better.

A pointer 150, 152 can be separately coupled to other parts of the apparatus such as a case 110, 120 and/or a connector 140. Alternatively, a pointer 150, 152 can be an integral part of a case 110, 120 and/or a connector 140 so that the pointer 150, 152 and the case 110, 120 and/or the connector 140 together form a monolithic piece. Therefore, a pointer 150, 152 can be rigidly coupled to a case 110, 120 and/or a connector so that they are stationary with respect to each other. This may simplify and speed up alignment of the apparatus 100 with the head. A pointer 150, 152 can be shaped or comprise a part shaped, for example, as a tip or a hole, adapted to be directed at an anatomical landmark.

When the apparatus comprises a connector 140 as described above, a pointer 152 may be arranged to extend 142 from the connector 140. In particular, a nasal pointer may extend from the connector 140 towards the nose of the head or all the way to the nose. Such a pointer 152 extending from the connector 140 can be adapted to be supported on the head, for example on the forehead. For this purpose, the pointer 152 may comprise an arm 154 which may be separately coupled to a head of the pointer 152 adapted to indicate the alignment of the apparatus 100. Alternatively, the arm 154 may be an integral part of the pointer 152 so that the pointer 152 and the arm 154 together form a monolithic piece. The arm 154 may be adapted to support the apparatus 100 also when the test subject is in a horizontal position.

As an alternative or an addition to aligning the apparatus using one or more pointers 150, 152, one or more imaging devices may be used. The apparatus 100 may therefore be also adapted to be aligned, wholly or partially, with internal or external imaging devices. The apparatus 100 may be adapted to transmit calibration information to an imaging device. It may also comprise one or more indicators of calibration information such as a visual indicator.

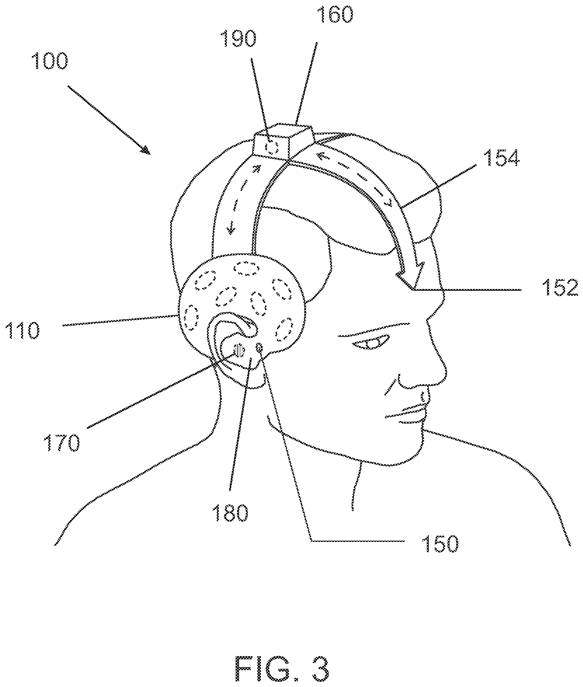

Figure 3 shows yet another example of the apparatus 100, where the apparatus 100 is mounted on the head of a test subject 200. The one or more of the features described herein can be used also in conjunction with the other examples.

Also in the figure, no wired output interface 130 is pictured. The apparatus 100 may still comprise a wireless output interface 130 for transmitting measurement data and/or receiving control instructions for the apparatus 100. Various examples for wirelessly transmitting data, such as a radio transmitter and/or receiver, are known to be a person skilled in the art and they can be used in the present case provided they do not cause excess interference with the measurement. To prevent interference, the apparatus 100 may comprise shielding, for example in the cases 110, 120.

The apparatus 100 may comprise various types of sensors or control circuits. These may include a processor configured to control the apparatus 100 and/or process measurement data. For these purposes, the apparatus 100 may also comprise a memory which may comprise program instructions for performing any or all of the functions of the processor. The apparatus 100 also comprise an accelerometer 190 for measuring acceleration of the apparatus 100 in one or more dimensions, for example in all three spatial dimensions. The accelerometer 190 can be configured to measure the acceleration in said dimensions to provide information of the movement of the apparatus 100, and consequently the head, and the apparatus 100 can be configured to process this information itself, for example in the processor, and/or the apparatus can be configured to transmit the information from the apparatus 100, for example through the output interface 130.

The apparatus 100 may comprise housing for any or all of the sensors and control circuits, such as the accelerometer 190. For example, they can be housed in either or both of the cases 110, 120 or the apparatus 100 can comprise one or more separate housings 160 for them. A housing 160 may be located on the connector 140, for example on the top of the connector 140, as pictured. As an example, an accelerometer 190 can be arranged in either or both of the cases 110, 120. Alternatively or additionally, one or more accelerometers 190 can be arranged in a separate housing 160, for example positioned on the connector 140.

The auditory stimuli can be introduced into the ear of the test subject from the apparatus 100 or from an external source. The apparatus 100 can also comprise one or more speakers 170 configured to direct auditory stimuli into either or both of the ears of the test subject. The one or more speakers 170 can be coupled with either or both of the cases 110, 120. They can also be housed in either or both of the cases 110, 120. The one or more speakers 170 may be adapted to be positioned on the ear canal to allow the auditory stimuli generated by them to be directly channeled into the ear canal. As an example, a case 110, 120 may extend, monolithically or with a separate extension, on the ear canal so that a speaker 170 may be coupled to the case 110, 120, or be housed in the case, on the ear canal. For this purpose, the apparatus 100 may comprise a tongue 180 extending from the case 110, 120. The tongue 180 may form a monolithic part of the corresponding case 110, 120. The tongue 180 may be adapted to extend on the ear channel from the front side of the ear. For example this way, the tongue 180 may be adapted to expose the helix of the ear of the test subject, while still extending over the ear channel. The tongue 180 may comprise means, for example an extension, to hold the ear open. These means can be adapted, for example, to abut the tragus to prevent it from closing on the ear channel. As one example, the apparatus 100 may comprise two speakers 170 where one is adapted to introduce auditory stimuli into the right ear of the test subject and the other is adapted to introduce auditory stimuli into the left ear of the test subject. The two speakers 170 may be adapted to allow the auditory stimuli to be separately introduced so that the apparatus 100 can be used to separately measure brain response when an auditory stimulus is introduced into the right ear and when an auditory stimulus is introduced into the left ear.

As an alternative or an addition to the one or more speakers 170, the apparatus 100 may also be adapted for one or more ducts for directing auditory stimuli into one or both ears of the test subject. The one or more ducts can be part of the apparatus 100 or the apparatus may comprise one or more brackets for external ducts. Using ducts allows the auditory stimuli to be generated remote from the ears so that the device generating the stimuli, such as the speaker 170, does not interfere electrically or in any other way with the measurement or the apparatus 100. As an example, the apparatus 100 may be adapted to use two ducts, where one is adapted to channel auditory stimuli into the right ear of the test subject and the other is adapted to channel auditory stimuli into the left ear of the test subject. The length of a duct may be a few centimeters or more, e.g. 5 centimeters or more and it can easily be even larger than a meter.

It is noted that when auditory stimuli is directed to an ear by the apparatus 100, for example from a speaker or from a duct, the auditory stimuli can also correspond to an audio track, such as music and/or an audio track of a movie, for relaxing the test subject.

In the figure, one type of a pointer 150 is illustrated. This pointer 150 comprises a hole for indicating the position of an anatomical landmark of the head of the test subject. The hole may be arranged on a tongue 180 as described above. This way the tongue 180 may function both as a positioning element and as a support for a speaker 170.

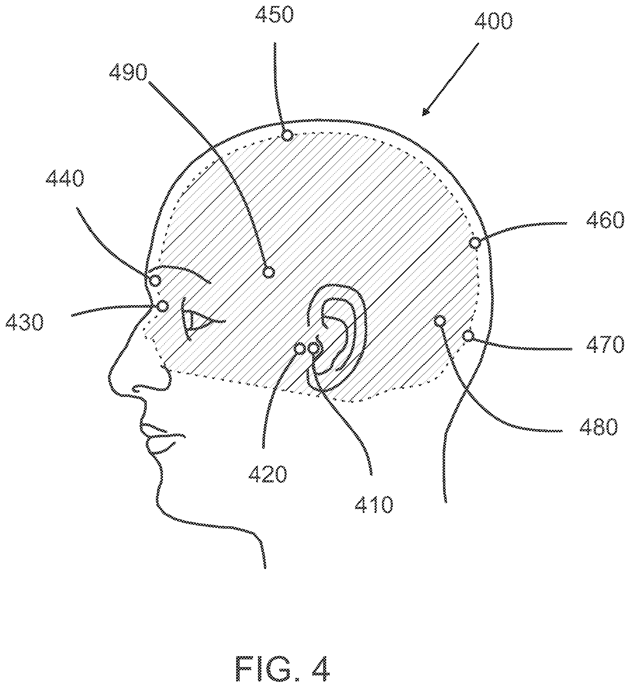

Figure 4 shows anatomical landmarks of a human head 400 in a side view. Some or all of these landmarks can be used in conjunction with the present invention, in particular for aligning the apparatus 100 with the head of a test subject. The apparatus 100 may comprise one or more pointers 150, 152 adapted to indicate position of an anatomical landmark of the head.

The illustrated examples for landmarks, which can be used for alignment of the apparatus 100 with respect to the head of a test subject are auricular point 410, pre-auricular point 420, nasion 430, glabella 440, bregma 450, lambda 460, inion 470, asterion 480 and pterion 490. Any combination of these may be used for alignment. However, it has been noticed that the auricular point 410 and, in particular, the pre-auricular point 420 can be used for effective positioning of the apparatus 100. A pointer 150 corresponding one or both of these points can be arranged as a part of a case 110, 120 or extending from the case 110, 120. This allows a convenient way to position the apparatus 100 in the plane formed by the surface of the head. On the other hand, nasion 430 has been found to be particularly effective in providing ease of use and accuracy in determining the orientation of the apparatus 100, in particular the pitch, with respect to the head of the test subject. For this purpose, a pointer 152 corresponding the nasion 430 can be arranged as a part of the apparatus 100, for example extending from a connector 140 between the cases 110, 120. This pointer 152 may be arranged to extend on the forehead and it may be of adjustable length to allow the pointer 152 to be used for different sized heads. The apparatus may comprise a detector for detecting the length of the pointer 152 or the pointer 152 may comprise a scale so that the length can be determined by an operator of the apparatus 100.

In some cases, the apparatus 100 may be extended to comprise sensors directed also to other parts of the brain in the vicinity of the auditory cortex. For example, one or both of the cases 110, 120 may be extended for this purpose. For example, the apparatus 100 may be adapted to measure also signals corresponding to somato-motor function and/or language lateralization for the brain. For this purpose, the apparatus 100 may be adapted to measure brain signals corresponding to the Broca's and/or Wernicke's areas, e.g. for localization of said area or areas. Correspondingly, the apparatus 100 may be adapted to cover also the Broca's and/or Wernicke's areas. The apparatus 100 may comprise one or more additional OPM sensors 112, 122 to be positioned on these areas during measurement. These additional extensions can be implemented as supplementary features to the primary function of the apparatus 100 for measuring magnetic brain response to auditory stimuli and therefore do not interfere with the function and construction of said apparatus 100 as described above.

The apparatus 100, at least in part, may be implemented in software, hardware, application logic or a combination of software, hardware and application logic. The application logic, software or instruction set may be maintained on any one of various conventional computer-readable media. A "computer-readable medium" may be any media or means that can contain, store, communicate, propagate or transport the instructions for use by or in connection with an instruction execution system, apparatus, or device, such as a computer.

The different functions discussed herein may be performed in a different order and/or concurrently with each other.

Any range or value given herein may be extended or altered without losing the effect sought, unless indicated otherwise. Also any embodiment may be combined with another embodiment unless explicitly disallowed.

Although the subject matter has been described in language specific to structural features and/or acts, it is to be understood that the subject matter defined in the appended claims is not necessarily limited to the specific features or acts described above. Rather, the specific features and acts described above are disclosed as examples of implementing the claims and other equivalent features and acts are intended to be within the scope of the claims.

It will be understood that the benefits and advantages described above may relate to one embodiment or may relate to several embodiments. The embodiments are not limited to those that solve any or all of the stated problems or those that have any or all of the stated benefits and advantages. It will further be understood that reference to 'an' item may refer to one or more of those items.

The term 'comprising' is used herein to mean including the method, blocks or elements identified, but that such blocks or elements do not comprise an exclusive list and a method or apparatus may contain additional blocks or elements.

## Claims

1. Head-mountable apparatus (100) for measuring magnetic brain response to auditory stimuli, comprising:
- a first case (110) adapted to be mounted on the head on the right auditory cortex, the first case housing a first set of optically pumped magnetometers (112) for measuring magnetic brain response to auditory stimuli;
- a second case (120) adapted to be mounted on the head on the left auditory cortex, the second case housing a second set of optically pumped magnetometers (122) for measuring magnetic brain response to auditory stimuli; and
- at least one output interface (130) for transmitting measurement data from the apparatus.

2. Head-mountable apparatus (100) according to claim 1, wherein the first case (110) covers the right auditory cortex and the second case covers (120) the left auditory cortex.

3. Head-mountable apparatus (100) according to claim 1 or 2, comprising a connector (140) mechanically coupling the first case (110) to the second case (120) .

4. Head-mountable apparatus (100) according to claim 3, wherein the connector (140) is adapted to extend across the top of the head for supporting the weight of the first case (110) and the second case (120) when the head is upright.

5. Head-mountable apparatus (100) according to claim 3 or 4, wherein the connector (140) is flexible for pressing the first case (110) and the second case (120) against the head for measurement by optically pumped magnetometers (112, 122).

6. Head-mountable apparatus (100) according to any of claims 3-5, wherein the length of the connector (140) between the first case (110) and the second case (120) is adjustable.

7. Head-mountable apparatus (100) according to any of the preceding claims, comprising one or more pointers (150, 152) for aligning the apparatus (100) with respect to one or more anatomical landmarks of the head.

8. Head-mountable apparatus (100) according to claim 7, wherein the one or more anatomical landmarks comprise the nasion (430) and/or a pre-auricular point (420).

9. Head-mountable apparatus (100) according to claim 7 or 8, wherein the one or more pointers (150, 152) comprises a tip and/or a hole for locating the one or more landmarks.

10. Head-mountable apparatus (100) according to any of claims 7-9, wherein the first case (110) and/or the second case (120) forms at least one of the one or more pointers (150).

11. Head-mountable apparatus (100) according to any of claims 7-10, comprising a connector (140) mechanically coupling the first case (110) with the second case (120); wherein at least one of the one or more pointers (152) is coupled to the connector (140) and adapted to extend on the forehead of the head towards the nose of the head.

12. Head-mountable apparatus (100) according to claim 11, wherein the length of extension from the connector (140) of the at least one pointer (152) coupled to the connector is adjustable.

13. Head-mountable apparatus (100) according to any of the preceding claims, adapted to use one or more speakers (170) and/or one or more ducts for directing auditory stimuli into an ear of the head.

14. Head-mountable apparatus (100) according to any of the preceding claims, comprising one or more accelerometers (190) for measuring movement of the head.

15. Head-mountable apparatus (100) according to any of the preceding claims, wherein each of the first case (110) and the second case (120) comprises at least one optically pumped magnetometer (112, 122) adapted to function as a gradiometer for measuring magnetic brain response to auditory stimuli.

## Patentansprüche

1. Am Kopf zu befestigende Einrichtung (100) zum Messen einer magnetischen Gehirnreaktion auf auditive Stimuli, umfassend:
- ein erstes Gehäuse (110), das angepasst ist, um am Kopf am rechten auditiven Kortex befestigt zu werden, wobei in dem ersten Gehäuse ein erster Satz von optisch gepumpten Magnetometern (112) zum Messen einer magnetischen Gehirnreaktion auf auditive Stimuli untergebracht ist;
- ein zweites Gehäuse (120), das angepasst ist, um am Kopf am linken auditiven Kortex befestigt zu werden, wobei in dem zweiten Gehäuse ein zweiter Satz von optisch gepumpten Magnetometern (122) zum Messen einer magnetischen Gehirnreaktion auf auditive Stimuli untergebracht ist; und
- mindestens eine Ausgangsschnittstelle (130) zum Übertragen von Messdaten von der Einrichtung.

2. Am Kopf zu befestigende Einrichtung (100) nach Anspruch 1, wobei das erste Gehäuse (110) den rechten auditiven Kortex bedeckt und das zweite Gehäuse (120) den linken auditiven Kortex bedeckt.

3. Am Kopf zu befestigende Einrichtung (100) nach Anspruch 1 oder 2, umfassend ein Verbindungsstück (140), welches das erste Gehäuse (110) an das zweite Gehäuse (120) koppelt.

4. Am Kopf zu befestigende Einrichtung (100) nach Anspruch 3, wobei das Verbindungsstück (140) angepasst ist, um sich über die Kopfoberseiten zum Tragen des Gewichts des ersten Gehäuses (110) und des zweiten Gehäuses (120) zu erstrecken, wenn der Kopf aufrecht gehalten wird.

5. Am Kopf zu befestigende Einrichtung (100) nach Anspruch 3 oder 4, wobei das Verbindungsstück (140) zum Andrücken des ersten Gehäuses (110) und des zweiten Gehäuses (120) gegen den Kopf zum Messen durch optisch gepumpte Magnetometer (112, 122) flexibel ausgelegt ist.

6. Am Kopf zu befestigende Einrichtung (100) nach einem der Ansprüche 3-5, wobei die Länge des Verbindungsstücks (140) zwischen dem ersten Gehäuse (110) und dem zweiten Gehäuse (120) verstellbar ist.

7. Am Kopf zu befestigende Einrichtung (100) nach einem der vorstehenden Ansprüche, umfassend einen oder mehrere Zeiger (150, 152) zum Ausrichten der Einrichtung (100) bezüglich einem oder mehreren anatomischen Orientierungspunkten des Kopfes.

8. Am Kopf zu befestigende Einrichtung (100) nach Anspruch 7, wobei der eine oder die mehreren Orientierungspunkte den Nasenwurzelpunkt (430) und/oder einen präaurikulären Punkt (420) umfassen.

9. Am Kopf zu befestigende Einrichtung (100) nach Anspruch 7 oder 8, wobei der eine oder die mehreren Zeiger (150, 152) eine Spitze und/oder ein Loch zum Lokalisieren des einen oder der mehreren Orientierungspunkte umfassen.

10. Am Kopf zu befestigende Einrichtung (100) nach einem der Ansprüche 7-9, wobei das erste Gehäuse (110) und/oder das zweite Gehäuse (120) mindestens einen des einen oder der mehreren Orientierungspunkte (150) bildet.

11. Am Kopf zu befestigende Einrichtung (100) nach einem der Ansprüche 7-10, umfassend ein Verbindungsstück (140), welches das erste Gehäuse (110) an das zweite Gehäuse (120) koppelt; wobei mindestens einer des einen oder der mehreren Zeiger (152) an das Verbindungsstück (140) gekoppelt ist und angepasst ist, um sich von der Stirn des Kopfes in Richtung der Nase des Kopfes zu erstrecken.

12. Am Kopf zu befestigende Einrichtung (100) nach Anspruch 11, wobei die Länge der Erstreckung von dem Verbindungsstück (140) des mindestens einen Zeigers (152), der an das Verbindungsstück gekoppelt ist, verstellbar ist.

13. Am Kopf zu befestigende Einrichtung (100) nach einem der vorstehenden Ansprüche, die angepasst ist, um einen oder mehrere Lautsprecher (170) und/oder eine oder mehrere Leitungen zum Leiten von auditiven Stimuli in ein Ohr des Kopfes zu verwenden.

14. Am Kopf zu befestigende Einrichtung (100) nach einem der vorstehenden Ansprüche, umfassend einen oder mehrere Beschleunigungsmesser (190) zum Messen einer Kopfbewegung.

15. Am Kopf zu befestigende Einrichtung (100) nach einem der vorstehenden Ansprüche, wobei jedes des ersten Gehäuses (110) und des zweiten Gehäuses (120) mindestens ein optisch gepumptes Magnetometer (112, 122) umfasst, das angepasst ist, um als ein Gradiometer zum Messen einer magnetischen Gehirnreaktion auf auditive Stimuli zu fungieren.

## Revendications

1. Appareil pouvant être monté sur la tête (100) pour mesurer une réponse cérébrale magnétique à des stimuli auditifs, comprenant :
- un premier boîtier (110) apte à être monté sur la tête sur le cortex auditif droit, le premier boîtier contenant un premier ensemble de magnétomètres à pompage optique (112) pour mesurer une réponse cérébrale magnétique à des stimuli auditifs ;
- un deuxième boîtier (120) apte à être monté sur la tête sur le cortex auditif gauche, le deuxième boîtier contenant un deuxième ensemble de magnétomètres à pompage optique (122) pour mesurer une réponse cérébrale magnétique à des stimuli auditifs ; et
- au moins une surface de sortie (130) pour transmettre des données de mesure à partir de l'appareil.

2. Appareil pouvant être monté sur la tête (100) selon la revendication 1, dans lequel le premier boîtier (110) couvre le cortex auditif droit et le deuxième boîtier (120) couvre le cortex auditif gauche.

3. Appareil pouvant être monté sur la tête (100) selon la revendication 1 ou 2, comprenant une jonction (140) couplant mécaniquement le premier boîtier (110) au deuxième boîtier (120).

4. Appareil pouvant être monté sur la tête (100) selon la revendication 3, dans lequel la jonction (140) est apte à s'étendre à travers le dessus de la tête pour supporter le poids du premier boîtier (110) et du deuxième boîtier (120) lorsque la tête est droite.

5. Appareil pouvant être monté sur la tête (100) selon la revendication 3 ou 4, dans lequel la jonction (140) est flexible pour appuyer le premier boîtier (110) et le deuxième boîtier (120) contre la tête pour une mesure par des magnétomètres à pompage optique (112, 122).

6. Appareil pouvant être monté sur la tête (100) selon l'une quelconque des revendications 3 à 5, dans lequel la longueur de la jonction (140) entre le premier boîtier (110) et le deuxième boîtier (120) est ajustable.

7. Appareil pouvant être monté sur la tête (100) selon l'une quelconque des revendications précédentes, comprenant un ou plusieurs pointeurs (150, 152) pour aligner l'appareil (100) par rapport à un ou plusieurs points de repère anatomiques de la tête.

8. Appareil pouvant être monté sur la tête (100) selon la revendication 7, dans lequel les un ou plusieurs points de repère anatomiques comprennent le nasion (430) et/ou un point pré-auriculaire (420).

9. Appareil pouvant être monté sur la tête (100) selon la revendication 7 ou 8, dans lequel les un ou plusieurs pointeurs (150, 152) comprennent une extrémité et/ou un trou pour positionner les un ou plusieurs points de repère.

10. Appareil pouvant être monté sur la tête (100) selon l'une quelconque des revendications 7 à 9, dans lequel le premier boîtier (110) et/ou le deuxième boîtier (120) forment au moins un des un ou plusieurs pointeurs (150).

11. Appareil pouvant être monté sur la tête (100) selon l'une quelconque des revendications 7 à 10, comprenant une jonction (140) couplant mécaniquement le premier boîtier (110) au deuxième boîtier (120) ; dans lequel au moins un des un ou plusieurs pointeurs (152) est couplé à la jonction (140) et apte à s'étendre sur le front de la tête vers le nez de la tête.

12. Appareil pouvant être monté sur la tête (100) selon la revendication 11, dans lequel la longueur d'extension à partir de la jonction (140) de l'au moins un pointeur (152) couplé à la jonction est ajustable.

13. Appareil pouvant être monté sur la tête (100) selon l'une quelconque des revendications précédentes, apte à utiliser un ou plusieurs haut-parleurs (170) et/ou un ou plusieurs conduits pour diriger des stimuli auditifs dans une oreille de la tête.

14. Appareil pouvant être monté sur la tête (100) selon l'une quelconque des revendications précédentes, comprenant un ou plusieurs accéléromètres (190) pour mesurer un mouvement de la tête.

15. Appareil pouvant être monté sur la tête (100) selon l'une quelconque des revendications précédentes, dans lequel chacun du premier boîtier (110) et du deuxième boîtier (120) comprend au moins un magnétomètre à pompage optique (112, 122) apte à servir de gradiomètre pour mesurer une réponse cérébrale magnétique à des stimuli auditifs.
